# DEMANDE DE BREVET EUROPEEN

(11) **EP 1 106 616 A2**
(43) Date de publication de la demande: **13.06.2001**
(21) Numéro de dépôt: 00403223.1
(22) Date de dépôt: 17.11.2000
(51) Int. Cl.: C07D 493/04, C10L 1/18, C08G 65/08

(54) **Dérivés de l'isosorbide utilisables dans des compositions détergentes pour carburants de type essence**

(30) Priorité: 08.12.1999 FR 9915572
(71) Demandeur: INSTITUT FRANCAIS DU PETROLE, 92852 Rueil-Malmaison Cedex (FR)
(72) Inventeur: Delfort, Bruno, 75005 Paris (FR); Joly, Stéphane, 78380 Bougival (FR); Lacome, Thierry, 92380 Garches (FR); Gateau, Patrick, 78310 Maurepas (FR); Paille, Fabrice, 78520 Limay (FR)

(57) **Abrégé**

Des compositions comprenant au moins un composé répondant à la formule générale :
dans laquelle R₁, R₂, R₃ et R₄ représentent chacun un atome d'hydrogène ou un 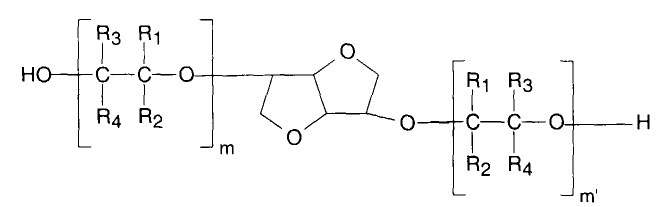 radical hydrocarboné, par exemple alkyle, de 1 à 30 atomes de carbone, l'un au moins des R₁, R₂, R₃ et R₄ étant un radical hydrocarboné, m et m' sont chacun un nombre de 1 à 30, avec m + m' de 4 à 60, peuvent être préparées par réaction de l'isosorbide avec un ou plusieurs composés présentant une fonction époxyde. Ces compositions peuvent être utilisées comme additifs détergents pour les carburants de type essence.

## Description

L'invention concerne de nouveaux composés utilisables seuls ou en mélanges dans des compositions d'additifs détergents pour carburants.

On sait que les moteurs d'automobiles ont tendance à former des dépôts à la surface des éléments du moteur en particulier sur les orifices du carburateur, le corps des papillons, les injecteurs de carburant, les orifices et les soupapes d'admission, en raison de l'oxydation et de la polymérisation de divers constituants hydrocarbonés du carburant. Ces dépôts, même lorsqu'ils ne sont présents qu'en faibles quantités, sont souvent responsables de problèmes de conduite importants, tels que le calage du moteur et des défaillances à l'accélération. De plus, les dépôts dans le moteur peuvent accroître de manière importante la consommation de carburant et la production d'émissions polluantes. C'est pourquoi le développement d'additifs détergents efficaces pour réguler ces dépôts revêt une importance considérable et a déjà fait l'objet de nombreuses recherches.

On a maintenant découvert une nouvelle famille de composés qui présentent une bonne efficacité comme additifs destinés à réduire les dépôts sur les injecteurs et sur les soupapes d'admission.

Les composés de l'invention peuvent être définis par la formule générale (I) suivante : 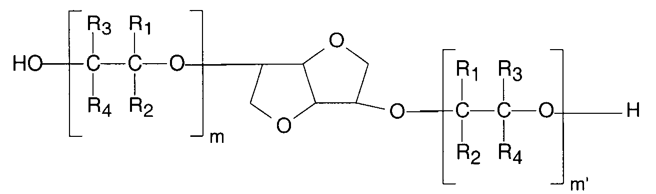 dans laquelle R₁, R₂, R₃ et R₄ représentent chacun un atome d'hydrogène ou un radical hydrocarboné, par exemple alkyle de 1 à 30 atomes de carbone l'un au moins des R₁, R₂, R₃ et R₄ étant un radical hydrocarboné ; m et m' sont chacun un nombre de 1 à 30, de préférence de 1 à 20, avec m + m' de 4 à 60, de préférence de 5 à 30.

Dans la même formule (I), les enchaînements 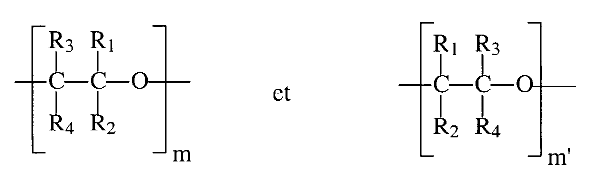 peuvent être constitués respectivement de motifs 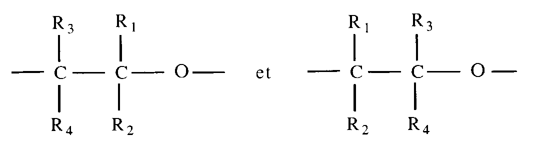 qui diffèrent entre eux par la nature de R₁, R₂, R₃ et/ou R₄.

Les composés de l'invention se présentent en général sous la forme de mélanges de composés qui diffèrent les uns des autres par la valeur de m et/ou m' et/ou par la nature des radicaux R₁, R₂, R₃ et R₄. Plutôt que de composés, on peut alors parler de compositions.

La synthèse des composés ou des compositions défini(e)s ci-dessus peut être effectuée comme décrit ci-après.

On fait réagir l'isosorbide de formule : 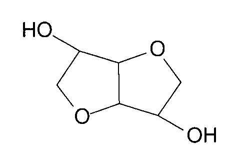 avec un ou plusieurs composés présentant une fonction époxyde, de formule générale : 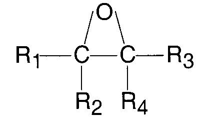 dans laquelle R₁, R₂, R₃ et R₄, représentent chacun un atome d'hydrogène ou un radical hydrocarboné, par exemple alkyle de 1 à 30 atomes de carbone, l'un au moins des R₁, R₂, R₃ et R₄ étant un radical hydrocarboné.

Les étapes de la préparation sont en général les suivantes :
a) dans un premier temps, on transforme l'isosorbide en alcoolate de métal alcalin (par exemple sodium ou potassium), par réaction avec un hydroxyde alcalin (par exemple soude ou potasse), un hydrure alcalin ou tout autre composé susceptible de former un alcoolate avec l'isosorbide ;
b) dans un deuxième temps, l'alcoolate alcalin amorce la polymérisation du ou des époxyde(s) par ouverture de ses (leurs) cycles ; et
c) dans un troisième temps, on traite le polyéther à terminaisons alcoolates formé, par de l'eau, un acide ou tout autre composé à hydrogène mobile, de manière à transformer les terminaisons alcoolates en fonctions alcools.

On décrit ci-après, à titre d'illustration, un mode opératoire particulier, étant entendu que la préparation des composés et des compositions de l'invention peut être effectuée par tout autre mode équivalent.

L'isosorbide est mélangé par exemple à de l'hydrure de sodium (à raison par exemple d'environ 0,5 % en poids). Après avoir purgé, sous agitation, le réacteur de l'hydrogène dégagé, on introduit un époxyde ou un mélange d'époxydes en une quantité calculée pour obtenir la valeur désirée de m+m', à une température de 80 à 180 °C et on maintient le mélange réactionnel à cette température jusqu'à la fin de la consommation du (ou des) époxyde(s). Après retour à la température ambiante, on dilue le milieu avec un solvant organique, par exemple hydrocarboné, tel que l'heptane, on le lave à l'eau une ou plusieurs fois, puis, après évaporation sous pression réduite de la phase organique, on obtient le produit recherché qui répond à la formule générale (l) :

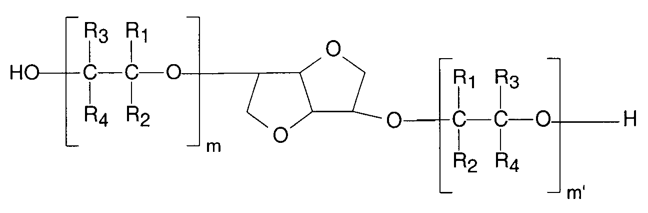

Les compositions de l'invention sont utilisables comme additifs détergents dans les carburants de type essence. Dans cette application, elles peuvent être ajoutées aux carburants à des concentrations par exemple de 20 à 5000 mg/litre. Elles peuvent être aussi utilisées en mélange avec tout autre composé détergent.

Les exemples suivants illustrent l'invention sans en limiter la portée.

### Exemple 1

Dans un réacteur permettant d'opérer sous pression, équipé d'un système d'agitation, d'un système d'introduction de réactifs, d'un système de mesure de la température et de la pression, on introduit 43,8 g (0,3 mole) d'isosorbide et 0,3 g d'hydrure de sodium. Après avoir purgé, sous agitation, le réacteur de l'hydrogène dégagé, on introduit 300 g (4,16 moles) de 1,2-époxybutane. On porte progressivement le milieu à la température de 145 °C et on le maintient à cette température jusqu'à ce que la chute de pression indique la fin de la consommation de 1,2-époxybutane. Après retour à la température ambiante, le milieu est dilué avec de l'heptane, lavé avec 2 fois 100 g d'eau, puis, après évaporation sous pression réduite de la phase organique, on obtient 337 g d'un liquide limpide jaune pâle, soluble dans l'essence et dont la structure correspond à la formule générale : 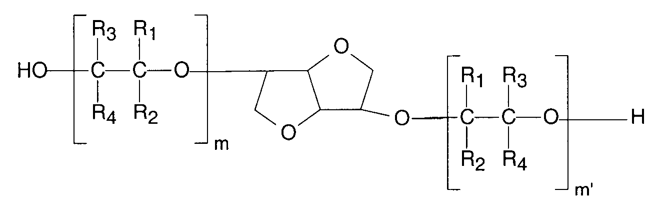 dans laquelle R₁ = R₂ = R₃ = H ; R₄ = -CH₂-CH₃ ; et (m + m') _{moyen} = 14.

### Exemple 2

Dans un réacteur permettant d'opérer sous pression, équipé d'un système d'agitation, d'un système d'introduction de réactifs, d'un système de mesure de la température et de la pression, on introduit 43,8 g d'isosorbide (0,3 mole) et 0,3 g d'hydrure de sodium. Après avoir purgé, sous agitation, le réacteur de l'hydrogène dégagé, on introduit 420 g (5,8 moles) de 1,2-époxybutane. On porte progressivement le milieu à la température de 145 °C et on le maintient à cette température jusqu'à ce que la chute de pression indique la fin de la consommation de 1,2-époxybutane. Après retour à la température ambiante, le milieu est dilué avec de l'heptane, lavé avec 2 fois 100 g d'eau, puis, après évaporation sous pression réduite de la phase organique, on obtient 453 g d'un liquide limpide jaune pâle, soluble dans l'essence et dont la structure correspond à la formule générale : 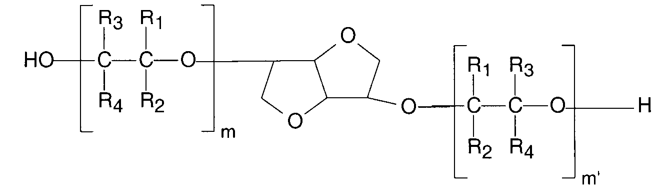 dans laquelle R₁ = R₂ = R₃ = H ; R₄ = -CH₂-CH₃ ; et (m+m') _{moyen} = 19.

### Exemple 3

Dans un réacteur permettant d'opérer sous pression, équipé d'un système d'agitation, d'un système d'introduction de réactifs, d'un système de mesure de la température et de la pression, on introduit 43,8 g d'isosorbide (0,3 mole) et 0,3 g d'hydrure de sodium. Après avoir purgé, sous agitation, le réacteur de l'hydrogène dégagé, on introduit 300 g (5,2 moles) de 1,2-époxypropane. On porte progressivement le milieu à la température de 145 °C et on le maintient à cette température jusqu'à ce que la chute de pression indique la fin de la consommation de 1,2-époxypropane. Après retour à la température ambiante, le milieu est dilué avec de l'heptane, lavé avec 2 fois 100 g d'eau, puis, après évaporation sous pression réduite de la phase organique, on obtient 335 g d'un liquide limpide jaune pâle, soluble dans l'essence et dont la structure correspond à la formule générale : 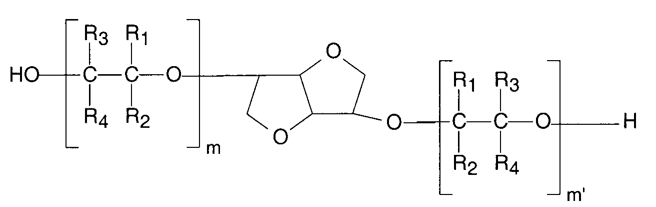 dans laquelle R₁ = R₂ = R₃ = H ; R₄ = -CH₃ ; et (m+m') _{moyen} = 17.

### Exemple 4

Dans un réacteur permettant d'opérer sous pression, équipé d'un système d'agitation, d'un système d'introduction de réactifs, d'un système de mesure de la température et de la pression, on introduit 43,8 g d'isosorbide (0,3 mole) et 0,3 g d'hydrure de sodium. Après avoir purgé, sous agitation, le réacteur de l'hydrogène dégagé, on introduit 210 g (2,9 mole) de 1,2 époxybutane et 90 g de 1,2 époxydodécane (0,48 mole). On porte progressivement le milieu à la température de 145 °C et on le maintient à cette température pendant 5 heures. Après retour à la température ambiante, le milieu est dilué avec de l'heptane, lavé avec 2 fois 100 g d'eau, puis, après évaporation sous pression réduite de la phase organique, on obtient 333 g d'un liquide limpide jaune pâle, soluble dans l'essence et dont la structure correspond à la formule générale : 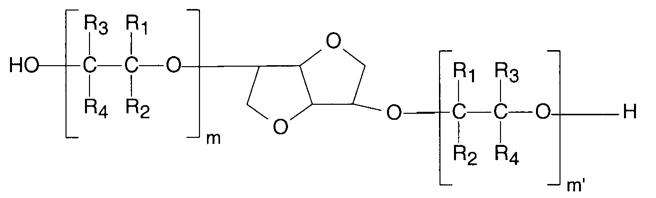 dans laquelle R₁ = R₂ = R₃ = H ; R₄ = -CH₂-CH₃ ou -(CH₂)₉-CH₃ ; et (m+m') _{moyen} = 14,6

### Exemple 5

Les produits préparés dans les exemples précédents 1 et 4 sont évalués comme additifs pour leurs propriétés détergentes à une concentration de 400 mg/litre dans une essence sans plomb d'indice d'octane "Recherche" de 96,8 et dont les caractéristiques figurent dans le tableau 1 suivant.

**TABLEAU 1**

| | | |
|---|---|---|
| Masse volumique à 15 °C | | 753,9 kg/m3 |
| Pression de Vapeur Reid | | 60,2 kPa |
| Teneur en plomb | | < 2 mg/l |
| Distillation | | |
| | Point initial | 33,0 °C |
| | 5 % | 45,9 °C |
| | 10 % | 51,2 °C |
| | 20 % | 61,1 °C |
| | 30 % | 73,1 °C |
| | 40 % | 88,3 °C |
| | 50 % | 104,2 °C |
| | 60 % | 116,0 °C |
| | 70 % | 125,6 °C |
| | 80 % | 138,5 °C |
| | 90 % | 155,6 °C |
| | 95 % | 169,8 °C |
| | Point final | 189,0 °C |

L'évaluation est effectuée au moyen d'un essai sur moteur Mercedes M102E selon la méthode CEC-F-05-A-93. La durée de cet essai est de 60 heures. Cette méthode permet d'évaluer la quantité de dépôts formés sur les soupapes d'admission du moteur.

Les résultats qui figurent dans le tableau 2 suivant montrent l'effet des produits de l'invention pour réduire les dépôts sur les soupapes d'admission.

**TABLEAU 2**

| Additif | Teneur (mg/l) | Masse de dépôt sur les soupapes d'admission (mg) | | | | |
|---|---|---|---|---|---|---|
| | | Soupape 1 | Soupape 2 | Soupape 3 | Soupape 4 | Moyenne |
| Néant | 0 | 241 | 275 | 272 | 312 | 275 |
| Produit de l'Exemple 1 | 400 | 54 | 31 | 33 | 55 | 43 |
| Produit de l'Exemple 4 | 400 | 13 | 16 | 17 | 4 | 12,5 |

## Revendications

1. Composé caractérisé en ce qu'il répond à la formule générale (l) : 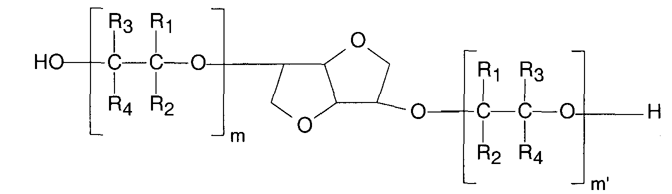 dans laquelle R₁, R₂, R₃ et R₄, représentent chacun un atome d'hydrogène ou un radical hydrocarboné l'un au moins des R₁, R₂, R₃ et R₄ étant un radical hydrocarboné ; m et m' sont chacun un nombre de 1 à 30, avec m + m' de 4 à 60.

2. Composé selon la revendication 1 caractérisé en ce que, dans la formule générale (I), les enchaînements 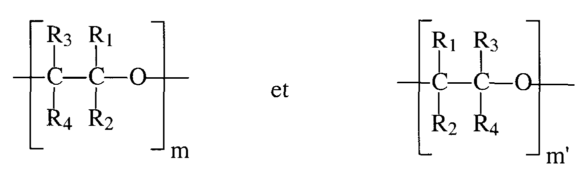 sont constitués respectivement de motifs 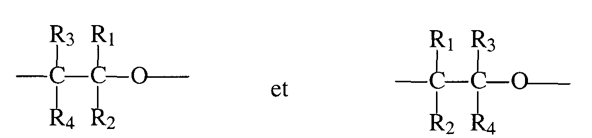 qui diffèrent entre eux par la nature de R₁, R₂, R₃ et/ou R₄.

3. Composé selon la revendication 1 ou 2 caractérisé en ce que, dans la formule générale (I), m et m' sont chacun un nombre de 1 à 20, avec m + m' de 5 à 30.

4. Composé selon l'une des revendications 1 à 3 caractérisé en ce que dans la formule générale (I), les radicaux hydrocarbonés R₁, R₂, R₃, et R₄ sont des radicaux alkyles de 1 à 30 atomes de carbone.

5. Composition caractérisée en ce qu'elle consiste en un mélange d'au moins deux composés selon l'une des revendications 1 à 4 qui diffèrent entre eux par les valeurs de m et/ou de m' et/ou par la nature d'au moins un des radicaux R₁ à R₄.

6. Procédé de préparation d'un composé selon l'une des revendications 1 à 4 ou d'une composition selon la revendication 5 caractérisé en ce que l'on fait réagir l'isosorbide de formule : 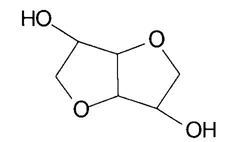 avec un ou plusieurs composés présentant une fonction époxyde et répondant à la formule générale : 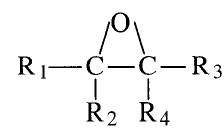 dans laquelle R₁, R₂, R₃ et R₄ sont définis comme dans l'une des revendications 1 à 4.

7. Procédé selon la revendication 6 caractérisé en ce que :
a) dans un premier temps, on transforme l'isosorbide en alcoolate de métal alcalin par réaction avec un hydroxyde alcalin un hydrure alcalin ou tout autre composé susceptible de former un alcoolate avec l'isosorbide ;
b) dans un deuxième temps, l'alcoolate alcalin amorce la polymérisation du (ou des) époxyde(s) par ouverture de ses (leurs) cycles ;
c) dans un troisième temps, on traite le polyéther à terminaisons alcoolates formé, par de l'eau, un acide faible ou tout autre composé à hydrogène mobile, de manière à transformer les terminaisons alcoolates en fonctions alcools.

8. Procédé selon la revendication 6 ou 7 caractérisé en ce que l'on mélange l'isosorbide à de l'hydrure de sodium ; on purge sous agitation le réacteur de l'hydrogène dégagé ; on introduit l'époxyde ou un mélange d'époxydes en une quantité calculée pour obtenir la valeur désirée de m+m', à une température de 80 à 180 °C et on maintient le mélange réactionnel à cette température jusqu'à la fin de la consommation du (ou des) époxyde(s); après retour à la température ambiante, on dilue le milieu avec un solvant organique, on le lave à l'eau une ou plusieurs fois, puis, après évaporation sous pression réduite de la phase organique, on obtient un composé selon l'une des revendications 1 à 4 ou une composition selon la revendication 5.

9. Utilisation d'un composé selon l'une des revendications 1 à 4 ou d'une composition selon la revendication 5 comme additif détergent dans une essence.

10. Utilisation selon la revendication 9 caractérisée en ce que ledit composé ou ladite composition est utilisé(e) en mélange avec tout autre composé détergent.
